(19) Europäisches Patentamt
European Patent Office
Office européen des brevets

(11) Publication number: 0 155 015
A2

(12) # EUROPEAN PATENT APPLICATION

(21) Application number: 85200118.9

(22) Date of filing: 05.02.85

(51) Int. Cl.[4]: A 61 K 7/08

(30) Priority: 09.02.84 JP 23579/84

(43) Date of publication of application: 18.09.85
Bulletin 85/38

(84) Designated Contracting States: CH DE FR LI

(71) Applicant: SHIONOGI & CO., LTD., 12,
Dosho-machi 3-chome Higashi-ku, Osaka 541 (JP)

(72) Inventor: Ukita, Hisako, 5-10-7, Uematsu-cho, Yao-shi Osaka (JP)
Inventor: Kawai, Sadao, 1-15-7, Kawai, Matsubara-shi Osaka (JP)
Inventor: Egawa, Shohei, 2-4-15, Nanatsumatsu-cho, Amagasaki-shi Hyogo (JP)

(74) Representative: Hranitzky, Wilheim Max et al, NOVAPAT - CABINET CHEREAU 5, Place du Molard, CH-1204 Genève (CH)

(54) **Less-irritant hair-rinse composition.**

(57) Less-irritant hair-rinse compositions available to even allergic persons susceptible to dermatitis or patients with atopic dermatitis comprise quaternary ammonium salt 1–5 w/w %, glycerin $C_8$–$C_{20}$ fatty acid ester 1–7 w/w %, $C_8$–$C_{20}$ alcohol 1–7 w/w/ %, and polyoxyethylene lanolin 0.1–3 w/w %.

EP 0 155 015 A2

# LESS-IRRITANT HAIR-RINSE COMPOSITION

Hair rinse compositions are, generally, used not only to give luster and softness to the hair after washing, but also to reduce the static electricity on the hair. Most of recent hair rinse compositions comprise quaternary ammonium salts as the main ingredient; the quaternary ammonium salts, however, are toxic as well as skin-irritating. Accordingly, patients with dermatitis have not been able to allowed to use hair rinses containing said ammonium salts.

The present inventors have made efforts to develop a hair rinse composition which can be used by persons with dermatitis as well as allergic persons susceptible to dermatitis, or patients with atopic dermatitis. They have found out that the addition of polyoxyethylene lanolin as a surfactant at a certain ratio to a rinse formulation in additon to a quaternary ammonium salt is effective to decrease the toxicity of said ammonium salt to give a practically irritation-free hair rinse composition; they finally have accomplished the present invention based on this finding.

The present invention relates to novel less-irritating hair rinse compositions, particularly hair rinse compositions containing polyoxyethylene lanolin, more specifically those compositions containing (a) a quaternary ammonium salt at 1-5 w/w %, (b) glycerol $C_8$-$C_{20}$ fatty acid ester at 1-7 w/w %, (c) a $C_8$-$C_{20}$ higher alcohol at 1-7 w/w % and (d) polyoxyethylene lanolin at 0.1-3 w/w %.

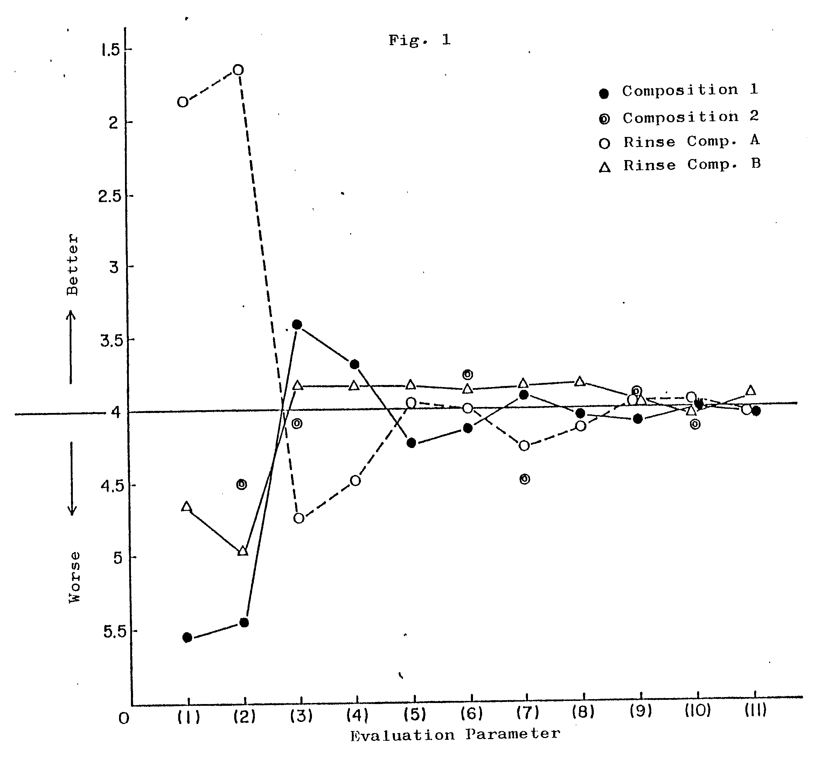

Figure 1 shows the result from organoleptic assessment through the usage of the rinse compositions 1 (●), and 2 (◎) according to this invention, along with the commercially available rinse compositions A (○) and B (△).

The present invention relates to less-irritating hair rinse compositions which contain (a) a quaternary ammonium salt at 1-5 w/w %, (b) glycerol $C_8$-$C_{20}$ fatty acid ester at 1-7 w/w %, (c) a $C_8$-$C_{20}$ higher alcohol at 1-7 w/w % and (d) polyoxyethylene lanolin at 0.1-3 w/w %.

Components of the hair rinse composition of the present invention and their relative amounts (by % weight) are as follows:

1. Quaternary ammonium salt: 1-5 w/w %
2. Glycerol $C_8$-$C_{20}$ fatty acid ester: 1-7 w/w %
3. $C_8$-$C_{20}$ Higher alcohol: 1-7 w/w %
4. Polyoxyethylene lanolin: 0.1-3 w/w %
5. Deionized water: a sufficient quantity to bring the

whole to 100 w/w %.

If desired, color additives, perfumes, preservatives, vitamins as hair growth stimulants, pH adjusters, and the like, may be formulated thereto in addition to the above components.

The quaternary ammonium salts specified in the present specification are the compounds generally used as cationic-type surfactants in the field of cosmetics, and are represented by the following formula:

$$\left[ R^1 - \overset{\displaystyle R^2}{\underset{\displaystyle CH_3}{N}} - CH_3 \right]^+ Cl^-$$

(wherein $R^1$ is $C_1$-$C_{20}$ alkyl or $C_8$-$C_{20}$ alkenyl, and $R^2$ is $C_8$-$C_{20}$ alkyl or $C_8$-$C_{20}$ alkenyl)
$R^1$ and $R^2$ include octyl, nonyl, decyl, undecyl, lauryl myristyl, cetyl, stearyl, oleyl, arachidyl and the like. Representatives of the applicable quaternary ammonium salts are, for example, didecyldimethylammonium chloride, dilauryldimethylammonium chloride, dicetyldimethylammonium chloride, distearyldimethylammonium chloride, stearyltrimethylammonium chloride, dicocodimethylammonium

chloride, and the like; most preferred is distearyldimethylammonium chloride. One or more of the substances described above may be formulated to the hair rinse composition at an amount of 1-5 w/w % to the whole.

A glycerol $C_8$-$C_{20}$ fatty acid ester means a mono- or di-ester of a fatty acid containing 8 to 20 carbon atoms with glycerin, and are commonly employed as an emulsifying agent in the cosmetic industry. The fatty acid containing 8 to 20 carbon atoms include, for example, caprylic acid, capric acid, lauric acid, myristic acid, palmitic acid, stearic acid, oleic acid, linoleic acid and the like. Monoesters of those fatty acids can be exemplified by glycerol mono-caprylate, glycerol mono-caprate, glycerol monolaurate, glycerol mono-myristate, glycerol mono-palmitate, glycerol mono-stearate, glycerol mono-isostearate, glycerol mono-oleate or the like. The di-esters are, for example, glycerol dilaurate, glycerol distearate, glycerol dioleate, glycerol stearate laurate, and the like, in particular a lipophilic glycerol mono-stearate is preferably employed. One or more of the above esters can be added at an amount of 1-7 w/w % of the whole hair rinse composition.

A $C_8$-$C_{20}$ alcohol means a straight or branch chained alcohol having 8 to 20 carbon atoms, and is usually employed as an oil phase component of detergents. Examples of such alcohols are lauryl alcohol, myristyl alcohol, cetyl

alcohol, stearyl alcohol, cetostearyl alcohol, oleyl alcohol, and the like. These alcohols can be used singly or in combinations. This ingredient is formulated at an amount of 1-7 w/w % to the whole hair rinse composition.

Polyoxyethylene lanolin (hereinafter referred to as POE-lanolin) is also called for polyethyleneglycol lanolin, which is a polyethyleneglycol derivative of lanolin and has commonly been used as a non-ionic surfactant. For the hair rinse compositions in accordance with the present invention, the use of said derivative having 10-30 molar ethylene oxide unit in the molecule provides an acceptable result. Typical examples of POE-lanolins are commercially available POE(10)-lanolin, POE(20)-lanolin, POE(24)-lanolin, POE(27)-lanolin, POE(30)-lanolin, and the like. Those lanolins are used singly or in combination with the others at a total amount of 0.1-3 w/w % to the whole rinse composition.

Additives, which may be employed in case of need, include dandruff removing agents, ultraviolet ray blocking agents, antihistamines, antioxidants, and the like, in addition to the aforementioned color additives, perfumes, preservatives, hair growth stimulants and pH adjusters. These additives can safely be chosen from those widely employed in cosmetics; particularly, no problems will be brought about by use of those additives allowed by Raw Material Standards for Cosmetics and by Drugs, Cosmetics,

and Medical Instruments Act. For example, benzoic acid, benzoates, salicylic acid, salicylates, sorbic acid, sorbates or parahydroxybenzoic acid esters may be employed as a preservative. For hair growth stimulants, vitamins (vitamins $B_2$, $B_6$, D, and E, pantothenic acid, and biotin), amino acids such as valine, leucine, threonine, lysine, methionine, phenylalanine, tryptophan, and the like, and proteins such as collagen, and the like, may be employed. For pH adjusters, an organic or inorganic compound such as citric acid, di- or tri-ethanolamine, sodium hydroxide or the like, may be employed as needed.

A sufficient quantity of deionized water is added to the mixture of aforementioned ingredients to bring the whole to 100 w/w %. A procedure of preparing of the hair rinse composition of the present invention is described below.

A mixture of a quaternary ammonium salt and a glycerol $C_8$-$C_{20}$ fatty acid ester is mixed with a $C_8$-$C_{20}$ higher alcohol and polyoxyethylene lanoline, and the whole is stirred while heating at about 70 to 80 °C. Water which has been warmed up to 70 to 80 °C is then gradually added to the mixture to give an emulsion. After completion of the emulsification, the resulting mixture is slowly cooled to around 35 °C. Vitamins, color additives and/or perfumes are added as needed. Then the mixture is adjusted to pH 4.0 or around. A sufficient quantity of deionized

water is then added to bring the whole to 100 w/w %. Finally, the composition is cooled to room temperature.

The rinse compositions according to the present invention can be prepared by the aforementioned method, wherein the order of adding each ingredient and the temperature to be applied may be changed in compliance with properties of the ingredient or depending on whether such additives should be added.

The rinse compositions of the present invention can stand comparison with commercially available rinse compositions with regard to comfortability. Furthermore, the compositions of the present invention can reduce the itching sensation of patients with atopic dermatitis by 80%, while the application of conventional rinse compositions usually cause exacerbation.

Figure 1 illustrates the test results concerning cosmetic performamce and comfortability of the rinse compositions 1 (●), and 2 (◎) of the present invention, and those of the commercially available rinse compositions A (○) and B (△). They were evaluated according to the following parameters.

(1) Ease of application of the rinse composition to the hair.

(2) Smoothness of the hair after application.

(3) Ease in rinsing, especially in the foaming off.

(4) Ease in rinsing, especially the sliminess caused by residue from the rinse.

(5) Pliability of the hair after towel-drying.

(6) Stiffness of the hair after towel-drying,

(7) Ease or speed of drying (evaluated 2 minutes after using a hair-drier),

(8) Ease in hair combing after use of the rinse.

(9) Pliability of the hair on combing.

(10) Stickness of the hair on combing.

(11) Overall evaluation as to cosmetic performance and comfortability.

The evaluation was carried out by the one-to-one comparison method concerning each of the above parameters, with a result that, as shown in Figure 1, there are no significant differences among the four. It is noteworthy that neither of the commercially available rinse compositions contain polyoxyethylene lanolin.

The rinse compositions 1 and 2 of the present invention and the commercially available rinse B were subjected to patch testing. The results are given below.

(1) TEST METHOD

To a commercially available Adhesive Plaster For Patch Test® (made by Torii Co.,Ltd) was applied 0.025 ml of a 5 % solution of each rinse. The patch was then placed on the inner side of the forearm (8 female subjects), and

removed after 24 hours. Skin irritation was judged according to the degree of erythema of the skin at 1 hour and 24 hours after removal of the patch.

(2) CRITERIA FOR JUDGEMENT

− no change

⊖ slightly rough (skin squama)

± slight erythema

(±) slight erythema but a marked skin squama or skin luster

(3) TEST RESULTS

| Time of Judgement | Composition Tested | − | ⊖ | ± | (±) |
|---|---|---|---|---|---|
| after 1 hour | Composition 1 | 6 | 2 | | |
| | Composition 2 | 7 | | 1 | |
| | Rinse Comp. B | 4 | 2 | 1 | 1 |
| after 24 hours | Composition 1 | 7 | 1 | | |
| | Composition 2 | 8 | | | |
| | Rinse Comp. B | 6 | 2 | | |

The composition of the present invention is further embodied in the following examples, which are not to limit the scope of the present invention.

EXAMPLE 1

| | | |
|---|---|---|
| (A) | Distearyldimethylammonium chloride: | 2 (g) |
| | Glycerol monostearate: | 2 |
| | Stearyl alcohol: | 1.5 |
| | Polyoxyethylene(30)-lanolin: | 0.5 |
| (B) | Deionized water: | proper quantity |
| | Ethyl para-hydroxybenzoate : | 0.2 |
| (C) | Pantothenol : | 0.1 |
| | DL-$\alpha$-Tocopherol acetate: | 0.01 |
| | Perfume: | 0.2 |
| | 0.01 % Solution of Red No.106: | 1.5 |
| (D) | Citric acid: | proper quantity |
| | Deionized water: | a sufficient quantity to bring the whole to 100 g |

To mixture (A) which has been warmed up to 70 to 80 °C while stirring is slowly added the hot (70 to 80 °C) solution (B). The mixture is stirred for a while and gently cooled to around 35 °C, to which the composition (C) is added, then the pH is adjusted with pH adjuster (D) at around pH 4.0. After the addition of a sufficient quantity

of deionized water to bring the whole to 100 g, the mixture is cooled to room temperature.

EXAMPLES 2 - 8

In the same manner as in Example 1, the following rinse compositions are obtained.

| Ingredient (w/w %) \ Example No. | 2 | 3 | 4 | 5 | 6 | 7 | 8 |
|---|---|---|---|---|---|---|---|
| Distearyldimethylammonium chloride | 5 | 5 | 2.5 | 2 | 5 | 5 | 2.5 |
| Glyceryl mono-stearate | 3 | | 4 | 2 | | 2 | |
| Glyceryl mono-oleate | | 2 | | | 3 | | 4 |
| Stearyl alcohol | 4 | 3 | 2.5 | | | | |
| Cetostearyl alcohol | | | | 1.5 | 4 | 5 | 2.5 |
| Polyoxyethylene(10)-lanolin | | 1.0 | | 0.5 | | | 1.5 |
| Polyoxyethylene(30)-lanolin | 1.0 | | 1.5 | | 1.0 | 1.0 | |
| Preservative | 0.15 | | | | | | |
| d-Panthenol | 0.1 | 0.5 | 1.0 | 0.5 | 0.7 | 0.3 | 0.5 |
| DL-$\alpha$-Tocopherol acetate | 0.01 | 0.05 | 0.1 | 0.5 | 0.2 | 0.3 | 0.5 |
| Color additive | A proper quantity | | | | | | |
| Perfume | A proper quantity | | | | | | |
| pH-Adjuster | A necessary amount to adjust to around pH 4.0 | | | | | | |
| Deionized water | A sufficient quantity to make 100 | | | | | | |

What is claimed is:

1. A less-irritating hair rinse composition which comprises:

(a) quaternary ammonium salt 1-5 w/w %,

(b) glycerin $C_8$- $C_{20}$ fatty acid ester 1-7 w/w %,

(c) $C_8$-$C_{20}$ higher alcohol 1-7 w/w % and,

(d) polyoxyethylene lanolin 0.1-3 w/w %.

2. A composition claimed in claim 1, wherein the quaternary ammonium salt is a compound represented by the following formula:

wherein $R^1$ is $C_1$-$C_{20}$ alkyl or $C_8$-$C_{20}$ alkenyl and $R^2$ is $C_8$-$C_{20}$ alkyl or $C_8$-$C_{20}$ alkenyl.

3. A composition claimed in claim 1, wherein the quaternary ammonium salt is a member selected from the group consisting of didecyldimethylammonium chloride, dilauryldimethylammonium chloride, dicetyldimethylammonium chloride, distearyldimethylammonium chloride, stearyltrimethylammonium chloride and dicocodimethylammonium chloride.

4. A composition claimed in claim 3, wherein the quaternary ammonium salt is distearyldimethylammonium chloride.

5. A composition claimed in claim 1, wherein the glycerol $C_8$-$C_{20}$ fatty acid ester is a member selected from the group consisting of glycerol monocaprylate, glycerol monocaprate, glycerol monolaurate, glycerol monomyristate, glycerol monopalmitate, glycerol monostearate, glycerol isostearate, glycerol monooleate, glycerol dilaurate, glycerol distearate, glycerol dioleate and glycerol stearate laurate.

6. A composition claimed in claim 1, wherein the glycerol $C_8$-$C_{20}$ fatty acid ester is a lipophilic glycerol monostearate.

7. A composition claimed in claim 1, wherein the $C_8$-$C_{20}$ alcohol is a member selected from the group consisting of lauryl alcohol, myristyl alcohol, cetyl alcohol, stearyl alcohol, cetostearyl alcohol and oleyl alcohol.

8. A composition claimed in claim 1, wherein the polyoxyethylene lanolin has 10-30 molar ethylene oxide units in its molecule.

9. A composition claimed in claim 1, wherein the polyoxyethylene lanolin is a member selected from the group consisting of POE(10)-lanolin, POE(20)-lanolin, POE(24)-lanolin, POE(27)-lanolin and POE(30)-lanolin.

Fig. 1
Composition 1
Composition 2
Rinse Comp. A
Rinse Comp. B
1.5
2
2.5
3
3.5
4
4.5
5
5.5
0
Better
Worse
(1)
(2)
(3)
(4)
(5)
(6)
(7)
(8)
(9)
(10)
(11)
Evaluation Parameter